# EUROPEAN PATENT APPLICATION

(11) **EP 1 364 925 A1**
(43) Date of publication of application: **26.11.2003**
(21) Application number: 01941024.0
(22) Date of filing: 13.06.2001
(51) Int. Cl.: C04B 12/02, A61L 27/12, A61K 6/06

(54) **CALCIUM PHOSPHATE CEMENT**

(30) Priority: 28.02.2001 JP 2001054277; 28.02.2001 JP 2001054278; 28.02.2001 JP 2001054279
(71) Applicant: MITSUBISHI MATERIALS CORPORATION, Chiyoda-ku, Tokyo 100-8117 (JP)
(72) Inventor: HIRANO, M., c/o Mitsubishi Materials Corp., Yokozemachi Chichibu-gen, Saitama-ken (JP); TAKEUCHI, H., c/o Mitsubishi Materials Corp., Tokyo 100-8117 (JP); ASAOKA, N., c/o Mitsubishi Materials Corp., Yokozemachi, Chichibu-gun, Saitama-ken (JP)
(74) Representative: Gille Hrabal Struck Neidlein Prop Roos
(86) International application number: JP0104998
(87) International publication number: WO02068358

(57) **Abstract**

The calcium phosphate cement of the present invention contains 0.03 to 2 wt% of sodium pyrophosphate and 3 to 35 wt% of dibasic calcium phosphate, wherein the remainder is at least one type selected from the hydration reaction product of dibasic calcium phosphate and α-tribasic calcium phosphate, tetrabasic calcium phosphate and α-tribasic calcium phosphate, and unavoidable impurities.

## Description

### TECHNICAL FIELD

The present invention relates to a calcium phosphate cement that can be used, for example, in treatments for reinforcing natural bone in the field of medicine, including oral surgery, and which is capable of forming a high-strength hardened material.

### BACKGROUND ART

In general, cement used in treatments for reinforcing natural bone is required to have the following characteristics:
(1) setting properties in which a slurry obtained by adding an aqueous hardening solution solidifies;
(2) hardening properties in which a solidified material hardens in the presence of moisture;
(3) sufficient hardened material strength that allows movement of the treated reinforced bone; and
(4) absorption replacement in which the hardened material is regenerated as vital bone.
Various types of cement used in treatments for reinforcing natural bone have been proposed in the past for use as a cement provided with these characteristics.

On the other hand, since a hardened material is considered a biologically foreign material, although it is desirable to obtain strength required for treated reinforced bone using as little hardened material as possible, in the cement used in treatments for reinforcing natural bone of the prior art, a sufficiently high strength was unable to be obtained for the hardened material.

Consequently, a reduction of the amount of hardened material used as treated reinforced bone was unable to be satisfactorily achieved, resulting in the strong need for the development of a cement used in treatments for reinforcing natural bone that allows for further improvement in the strength of the hardened material.

### DISCLOSURE OF INVENTION

As a result of conducting research to develop a calcium phosphate cement that enables improvement of the strength of the hardened material, the inventors of the present invention discovered a mixed composition containing 0.03 to 2 wt% of sodium pyrophosphate and 3 to 35 wt% of dibasic calcium phosphate, wherein the remainder is at least one type selected from the hydration reaction product of dibasic calcium phosphate and α-tribasic calcium phosphate, tetrabasic calcium phosphate and α-tribasic calcium phosphate, and unavoidable impurities. Research results were also obtained in which, when this mixed composition is used as a calcium phosphate cement, the fluidity of the paste formed by the addition of an aqueous hardening solution is even further improved by the action of the above sodium pyrophosphate, the entrapment of air bubbles is remarkably reduced, it becomes possible to form a dense hardened material, and the strength of the formed hardened material can be remarkably improved.

The calcium phosphate cement according to a first preferred embodiment of the present invention contains 0.03 to 0.5 wt% of sodium pyrophosphate, 3 to 10 wt% of dibasic calcium phosphate, and 10 to 25 wt% of tetrabasic calcium phosphate, wherein the remainder is α-tribasic calcium phosphate and unavoidable impurities.

The calcium phosphate cement according to a second preferred embodiment of the present invention contains 0.03 to 1.5 wt% of sodium pyrophosphate and 3 to 25 wt% of dibasic calcium phosphate, wherein the remainder is α-tribasic calcium phosphate and unavoidable impurities.

The calcium phosphate cement according to a third preferred embodiment of the present invention contains 0.3 to 2 wt% of sodium pyrophosphate and 5 to 35 wt% of dibasic calcium phosphate, wherein the remainder is tetrabasic calcium phosphate and unavoidable impurities.

The calcium phosphate cement according to a fourth preferred embodiment of the present invention contains 0.03 to 2.0 wt% of sodium pyrophosphate, 2 to 10 wt% of the hydration reaction product of dibasic calcium phosphate and α-tribasic calcium phosphate, 3 to 10 wt% of dibasic calcium phosphate, and 10 to 25 wt% of tetrabasic calcium phosphate, wherein the remainder is α-tribasic calcium phosphate and unavoidable impurities.

### PREFERRED EMBODIMENTS OF THE INVENTION

The basic composition of the calcium phosphate cement of the present invention contains 0.03 to 2 wt% of sodium pyrophosphate and 3 to 35 wt% of dibasic calcium phosphate, and the remainder is at least one type selected from the hydration reaction product of dibasic calcium phosphate and α-tribasic calcium phosphate, tetrabasic calcium phosphate, and α-tribasic calcium phosphate, and unavoidable impurities. The following provides an explanation of the reason for limiting the blended composition in the manner described above.

### (a) Sodium pyrophosphate

Sodium pyrophosphate enhances the fluidity of the paste that is formed by adding an aqueous hardening solution to the cement, inhibits the entrapment of air bubbles and enables the formation of a dense hardened material. As a result, it demonstrates the action of improving the strength of the hardened material. In order to adequately enhance this action, during production of the calcium phosphate cement, it is desirable to first mix the sodium pyrophosphate with the dibasic calcium phosphate, and then mix in the remaining components, namely, at least one component selected from the hydration reaction product of dibasic calcium phosphate and α-tribasic calcium phosphate, tetrabasic calcium phosphate, and α-tribasic calcium phosphate.

If the blending ratio of sodium pyrophosphate is less than 0.03 wt%, the above action is diminished, while if the blending ratio is greater than 2 wt%, a tendency appears in which the time required for hardening of the hardened material is prolonged. Accordingly, the blending ratio is preferably 0.03 to 2 wt%.

### (b) Dibasic calcium phosphate

Dibasic calcium phosphate has the action of promoting setting of the paste into a hardened material. If the blending ratio is less than 3 wt%, the action of promoting setting is diminished, while if the blending ratio exceeds 35 wt%, the paste hardening time becomes excessively short, thereby decreasing the ease of handling. Accordingly, the blending ratio is preferably 3 to 35 wt%.

Furthermore, an aqueous solution in which one type or two or more types selected from, for example, disodium succinate, sodium chondroitin sulfate, sodium lactate, sodium phosphate, sodium chloride, sodium hydrogen sulfite, and sodium pyrosulfite, are dissolved at a concentration of 40 wt% or less, or distilled water, can be used for the aqueous hardening solution that hardens the calcium phosphate cement of the present invention by mixing therewith.

Next, the following provides a more detailed explanation of the composition for each of the preferred embodiments for carrying out the present invention.

### Embodiment 1

The calcium phosphate cement pertaining to a first preferred embodiment for carrying out the invention contains 0.03 to 0.5 wt% of sodium pyrophosphate, 3 to 10 wt% of dibasic calcium phosphate, and 10 to 25 wt% of tetrabasic calcium phosphate, wherein the remainder is α-tribasic calcium phosphate and unavoidable impurities.

In Embodiment 1, the ratio of sodium pyrophosphate is preferably 0.03 to 0.5 wt%, and more preferably 0.1 to 0.3 wt%. The ratio of dibasic calcium phosphate is preferably 3 to 10 wt%, and more preferably 4 to 8 wt%. The reasons for this are the same as previously described.

Tetrabasic calcium phosphate has an action of promoting the ease of absorption and replacement by which the hardened material is regenerated to vital bone. However, if its ratio is less than 10 wt%, the above action is diminished, while if the ratio exceeds 25 wt%, the strength of the hardened material decreases. Thus, the ratio of tetrabasic calcium phosphate is preferably 10 to 25 wt%, and more preferably 12 to 20 wt%.

### Embodiment 2

The calcium phosphate cement pertaining to a preferred second embodiment for carrying out the present invention contains 0.03 to 1.5 wt% of sodium pyrophosphate and 3 to 25 wt% of dibasic calcium phosphate, wherein the remainder is α-tribasic calcium phosphate and unavoidable impurities.

In Embodiment 2, if the ratio of sodium pyrophosphate is less than 0.03 wt%, the above-mentioned action is diminished, while if the ratio exceeds 1.5 wt%, the time required for the hardened material to harden is prolonged. Thus, the ratio of sodium pyrophosphate is preferably 0.03 to 1.5 wt%, and more preferably 0.1 to 1.0 wt%.

If the ratio of dibasic calcium phosphate is less than 3 wt%, the desired action of promoting setting is unable to be attained, while if the ratio exceeds 25 wt%, hardening is excessively promoted resulting in a decrease in handling ease. Thus, the ratio of dibasic calcium phosphate is preferably 3 to 25 wt%, and more preferably 6 to 20 wt%.

### Embodiment 3

The calcium phosphate cement pertaining to a preferred third embodiment for carrying out the present invention contains 0.03 to 2 wt% of sodium pyrophosphate and 5 to 35 wt% of dibasic calcium phosphate, wherein the remainder is tetrabasic calcium phosphate and unavoidable impurities.

In Embodiment 3, if the ratio of sodium pyrophosphate is less than 0.03 wt%, the above action is diminished, while if that ratio exceeds 2 wt%, the hardening time of the hardened material is prolonged. Thus, the ratio is preferably 0.03 to 2 wt%, and more preferably 0.3 to 1.5 wt%.

If the ratio of dibasic calcium phosphate is less than 5 wt%, the desired action of promoting setting cannot be ensured, while if the ratio exceeds 35 wt%, the hardening time becomes excessively short causing a decrease in handling ease. Thus, the ratio is preferably 5 to 35 wt%, and more preferably 10 to 30 wt%.

### Embodiment 4

The calcium phosphate cement pertaining to a preferred fourth embodiment for carrying out the present invention contains 0.03 to 2.0 wt% of sodium pyrophosphate, 2 to 10 wt% of the hydration reaction product of dibasic calcium phosphate and α-tribasic calcium phosphate, 3 to 10 wt% of dibasic calcium phosphate, and 10 to 25 wt% of tetrabasic calcium phosphate, wherein the remainder is α-tribasic calcium phosphate and unavoidable impurities.

In Embodiment 4, if the ratio of sodium pyrophosphate is less than 0.03 wt%, the above action is diminished, while if that ratio exceeds 2 wt%, the hardening time of the hardened material is prolonged. Thus, the ratio is preferably 0.03 to 2 wt%, and more preferably 0.1 to 1.0 wt%.

If the ratio of dibasic calcium phosphate is less than 3 wt%, the desired action of promoting setting cannot be ensured, while if the ratio exceeds 10 wt%, the hardening time becomes excessively short causing a decrease in handling ease. Thus, the ratio is preferably 3 to 10 wt%, and more preferably 4 to 7 wt%.

If the ratio of the above hydration product is less than 2 wt%, the desired effect of improving the strength tends to not be obtained, while if the ratio exceeds 10 wt%, the fluidity of the slurry decreases resulting in a loss of handling ease. Thus, the ratio is preferably 2 to 10 wt%, and more preferably 3 to 7 wt%. This hydration reaction product can be manufactured by mixing dibasic calcium phosphate and α-tribasic calcium phosphate at a blending ratio of preferably 1:5 to 1:30, and more preferably 1:10 to 1:20, as the mass ratio, and water is added to the resulting mixture at a mass ratio of 1:0.2 to 1:0.5, and more preferably 1:0.3 to 1:0.4, followed by the hydration reaction, and crushing to a particle size of about 1 to 300 µm.

### TESTS

The following provides an explanation of preferred tests of the calcium phosphate cement according to the present invention. However, the present invention is not limited to any of these tests, and numerous variations are possible within the scope described in the claims.

### Example 1

α-tribasic calcium phosphate and tetrabasic calcium phosphate were prepared as indicated below. Commercially available products were used for the sodium pyrophosphate and dibasic calcium phosphate.

### (1) α-tribasic calcium phosphate

3 moles of calcium hydroxide were suspended in 10 liters of water followed by slowly dripping in a 40 wt% aqueous phosphoric acid solution comprising 2 moles of phosphoric acid diluted with water, while stirring, and allowing to stand for 1 day at room temperature following completion of dripping. Next, the suspension was dried using a dryer under conditions of holding at 110°C for 24 hours to form an aggregate, the above aggregate was baked by holding at 1400°C for 3 hours, and the baked product was crushed and sieved through a screen with a mesh size of 88 µm (mean particle size: 6.5 µm) to produce α-tribasic calcium phosphate having purity of 99.9 wt%.

### (2) Tetrabasic calcium phosphate

4 moles of calcium hydroxide were suspended in 10 liters of water followed by slowly dripping in a 40 wt% aqueous phosphoric acid solution comprising 2 moles of phosphoric acid diluted with water, while stirring, and were allowed to stand for 1 day at room temperature following completion of dripping. Next, the suspension was dried using a dryer under conditions of holding at 110°C for 24 hours to form an aggregate, this aggregate was pre-baked under conditions of holding at 900°C for 3 hours, the pre-baked aggregate was baked under conditions of holding at 1400°C for 3 hours in a state in which the pre-baked aggregate was uniformly crushed, and the baked product was crushed and sieved through a screen with a mesh size of 88 µm (mean particle size: 6.5 µm) to produce a mixed product in which the content ratio of tetrabasic calcium phosphate was 90.5 wt%, and the remainder was composed of a substantially unavoidable impurity in the form of hydroxyapatite. Furthermore, in this example, the above mixed product was used as tetrabasic calcium phosphate.

Next, using the α-tribasic calcium phosphate and tetrabasic calcium phosphate, obtained by the above methods in the form of raw material powders, as well as commercially available sodium pyrophosphate and dibasic calcium phosphate, the sodium pyrophosphate and dibasic calcium phosphate were first kneaded for 30 minutes with a kneading machine at the ratios indicated in Table 1 followed by blending these kneaded powders with the other raw material powders in the ratios shown in Table 1 to produce Tests A1 through A15. On the other hand, Comparative Tests A1 through A11 were respectively prepared in which sodium pyrophosphate was not blended.

An aqueous hardening solution containing 5 wt% of sodium chondroitin sulfate, 15 wt% of disodium succinate hexahydrate, and 0.3 wt% of sodium hydrogen sulfite, with the remainder consisting of water, was added to the cement at the mass ratio of cement:aqueous hardening solution = 3:1 followed by mixing to obtain a slurry for the purpose of evaluating the hardness of the hardened materials formed from Tests A1 through A15 and Comparative Tests A1 through A11. The slurries were allowed to set to form cylindrical solidified bodies having a diameter of 6 mm and height of 12 mm. These solidified bodies were then hardened by immersing in an aqueous solution (simulated body fluid) containing 142.0 mM Na⁺, 5.0 mM K⁺, 1.5 mM Mg²⁺, 2.5 mM Ca²⁺, 148.8 mM Cl⁻, 4.2 mM HCO³⁻, and 1.0 mM HPO₄²⁻ followed by measurement of compressive strength of the hardened material following a 5-day hardening treatment. The measurement results are shown in Table 1.

**Table 1**

| | | Blending ratio (wt%) | | | | Compressive strength (MPa) |
|---|---|---|---|---|---|---|
| | | Sodium pyrophosphate | Dibasic calcium phosphate | Tetrabasic calcium phosphate | α-tribasic calcium phosphate + impurities | |
| Tests | A1 | 0.03 | 5 | 18 | balance | 84 |
| | A2 | 0.1 | 5 | 18 | balance | 88 |
| | A3 | 0.2 | 5 | 18 | balance | 90 |
| | A4 | 0.3 | 5 | 18 | balance | 92 |
| | A5 | 0.4 | 5 | 18 | balance | 95 |
| | A6 | 0.5 | 5 | 18 | balance | 94 |
| | A7 | 0.2 | 3 | 18 | balance | 94 |
| | A8 | 0.2 | 4 | 18 | balance | 90 |
| | A9 | 0.2 | 8 | 18 | balance | 86 |
| | A10 | 0.2 | 10 | 18 | balance | 81 |
| | A11 | 0.2 | 5 | 10 | balance | 78 |
| | A12 | 0.2 | 5 | 12 | balance | 80 |
| | A13 | 0.2 | 5 | 15 | balance | 75 |
| | A14 | 0.2 | 5 | 20 | balance | 72 |
| | A15 | 0.2 | 5 | 25 | balance | 70 |
| Comparative Tests | A1 | - | 3 | 18 | balance | 65 |
| | A2 | - | 4 | 18 | balance | 63 |
| | A3 | - | 5 | 18 | balance | 58 |
| | A4 | - | 8 | 18 | balance | 52 |
| | A5 | - | 10 | 18 | balance | 51 |
| | A6 | - | 5 | 10 | balance | 64 |
| | A7 | - | 5 | 12 | balance | 62 |
| | A8 | - | 5 | 15 | balance | 60 |
| | A9 | - | 5 | 17 | balance | 58 |
| | A10 | - | 5 | 20 | balance | 55 |
| | A11 | - | 5 | 25 | balance | 50 |

Based on the results shown in Table 1, hardened bodies formed from Tests A1 through A15 that contained sodium pyrophosphate clearly demonstrated even higher strength in comparison with the hardened bodies formed from Comparative Tests A1 through A11 that did not contain sodium pyrophosphate.

### Example 2

α-tribasic calcium phosphate powder obtained in the same manner as Example 1, and commercially available sodium pyrophosphate and dibasic calcium phosphate were used.

To begin with, sodium pyrophosphate and dibasic calcium phosphate were first mixed in the ratios shown in Table 2, and were kneaded for 30 minutes with a kneading machine. Next, the α-tribasic calcium powder was blended with these mixed powders in the ratios shown in Table 2 followed by mixing to form a mixture and produce Tests B1 through B15. On the other hand, Comparative Tests B1 through B8 were respectively produced into which magnesium phosphate was not blended.

Moreover, cylindrical solidified bodies measuring 6 mm in diameter and 12 mm in height were formed in the same manner as Example 1 using these cements for the purpose of evaluating the hardness of the hardened bodies formed from Tests B1 through B15 and Comparative Tests B through B8. These solidified bodies were hardened by immersing for 5 days in the same simulated body fluid as Example 1 followed by measurement of compressive strength of the hardened bodies following a 5-day hardening treatment. The measurement results are shown in Table 2.

**Table 2**

| | | Blending ratio (wt%) | | | Compressive strength (MPa) |
|---|---|---|---|---|---|
| | | Sodium pyrophosphate | Dibasic calcium phosphate | α-tribasic calcium phosphate + impurities | |
| Tests | B1 | 0.03 | 12 | balance | 81 |
| | B2 | 0.1 | 12 | balance | 84 |
| | B3 | 0.2 | 12 | balance | 88 |
| | B4 | 0.3 | 12 | balance | 89 |
| | B5 | 0.4 | 12 | balance | 90 |
| | B6 | 0.5 | 12 | balance | 88 |
| | B7 | 1 | 12 | balance | 87 |
| | B8 | 1.5 | 12 | balance | 85 |
| | B9 | 0.3 | 3 | balance | 83 |
| | B10 | 0.3 | 5 | balance | 80 |
| | B11 | 0.3 | 7 | balance | 75 |
| | B12 | 0.3 | 10 | balance | 77 |
| | B13 | 0.3 | 16 | balance | 75 |
| | B14 | 0.3 | 20 | balance | 73 |
| | B15 | 0.3 | 25 | balance | 70 |
| Comparative Tests | B1 | - | 3 | balance | 64 |
| | B2 | - | 5 | balance | 60 |
| | B3 | - | 7 | balance | 60 |
| | B4 | - | 10 | balance | 57 |
| | B5 | - | 12 | balance | 56 |
| | B6 | - | 16 | balance | 51 |
| | B7 | - | 20 | balance | 49 |
| | B8 | - | 25 | balance | 44 |

As is clear from Table 2, the hardened bodies formed using Tests B1 through B15 that contained sodium pyrophosphate demonstrated even higher strength as compared with the hardened bodies formed from Comparative Tests B1 through B8 that did not contain sodium pyrophosphate.

### Example 3

Tetrabasic calcium phosphate powder was used that was produced according to the same method as Example 1. In addition, commercially available powders were respectively used for the sodium pyrophosphate and dibasic calcium phosphate. The sodium pyrophosphate powder and dibasic calcium phosphate powder were first kneaded for 30 minutes with a kneading machine at the ratios shown in Table 3, the tetrabasic calcium phosphate powder was blended into this mixed powder at the ratios indicated in Table 3 followed by mixing to obtain mixed compositions and produce Tests C1 through C14. In addition, Comparative Tests C1 through C7 were produced that did not contain sodium pyrophosphate.

Cylindrical solidified bodies measuring 6 mm in diameter and 12 mm in height were obtained according to the same method as Example 1 using Tests C1 through C14 and Comparative Tests C1 through C7. These solidified bodies were then hardened by immersing for 5 days in the same simulated body fluid as Example 1 followed by measurement of compressive strength of the hardened bodies after a 5-day hardening treatment. The measurement results are shown in Table 3.

**Table 3**

| | | Blending ratio (wt%) | | | Compressive strength (MPa) |
|---|---|---|---|---|---|
| | | Sodium pyrophosphate | Dibasic calcium phosphate | Tetrabasic calcium phosphate + impurities | |
| Tests | C1 | 0.03 | 15 | balance | 66 |
| | C2 | 0.3 | 15 | balance | 67 |
| | C3 | 0.6 | 15 | balance | 70 |
| | C4 | 0.9 | 15 | balance | 72 |
| | C5 | 1.2 | 15 | balance | 74 |
| | C6 | 1.5 | 15 | balance | 75 |
| | C7 | 1.8 | 15 | balance | 77 |
| | C8 | 2.0 | 15 | balance | 80 |
| | C9 | 0.7 | 5 | balance | 74 |
| | C10 | 0.7 | 10 | balance | 72 |
| | C11 | 0.7 | 15 | balance | 72 |
| | C12 | 0.7 | 25 | balance | 64 |
| | C13 | 0.7 | 30 | balance | 64 |
| | C14 | 0.7 | 35 | balance | 61 |
| Comparative Tests | C1 | - | 5 | balance | 57 |
| | C2 | - | 10 | balance | 55 |
| | C3 | - | 15 | balance | 53 |
| | C4 | - | 20 | balance | 50 |
| | C5 | - | 25 | balance | 49 |
| | C6 | - | 30 | balance | 45 |
| | C7 | - | 35 | balance | 42 |

As is clear from Table 3, the hardened bodies formed from Tests C1 through C14 that contained sodium pyrophosphate had even higher strength in comparison with the hardened bodies of Comparative Tests C1 through C7 that did not contain sodium pyrophosphate.

### Example 4

α-tribasic calcium phosphate and tetrabasic calcium phosphate powder were used that were produced according to the same method as Example 1. In addition, commercially available powders were respectively used for the sodium pyrophosphate and dibasic calcium phosphate. The hydration reaction product of the dibasic calcium phosphate and α-tribasic calcium phosphate was prepared in the manner described below.

Commercially available dibasic calcium phosphate and α-tribasic calcium phosphate prepared in the manner previously described were mixed at weight ratios of 1:15 and 1:30, respectively, and after hardening the mixtures by adding water at a weight ratio of 3:1, the hardened bodies were crushed to 300 µm or less to prepare two types of hydration reaction products. The mixture having a weight ratio of 1:15 is shown in Table 4 as C, while the mixture having a weight ratio of 1:30 is shown in Table 4 as F.

The sodium pyrophosphate powder and dibasic calcium phosphate powder were first mixed at the ratios shown in Table 4, kneaded for 30 minutes with a kneading machine, and the remaining raw materials were blended into this mixture at the ratios shown in Table 4 followed by mixing to obtain mixed compositions and produce Tests D1 through D15. In addition, Comparative Tests D1 through D5 were produced that did not contain sodium pyrophosphate.

Cylindrical solidified bodies measuring 6 mm in diameter and 12 mm in height were obtained according to the same method as Example 1 using Tests D1 through D15 and Comparative Tests D1 through D5. These solidified bodies were then hardened by immersing for 5 days in the same simulated body fluid as Example 1 followed by measurement of compressive strength of the hardened bodies after a 5-day hardening treatment. The measurement results are shown in Table 4.

**Table 4**

| | | Blending ratio (wt%) | | | | | Compressive strength (MPa) |
|---|---|---|---|---|---|---|---|
| | | Sodium pyrophosphate | Hydration reaction product | Tetrabasic calcium phosphate | Dibasic calcium phosphate | α-tribasic calcium phosphate | |
| Tests | D1 | 0.03 | F:5 | 18 | 5 | balance | 90 |
| | D2 | 0.1 | C:5 | 18 | 5 | balance | 89 |
| | D3 | 0.3 | C:5 | 18 | 5 | balance | 92 |
| | D4 | 0.5 | C:5 | 18 | 5 | balance | 95 |
| | D5 | 2 | C:5 | 18 | 5 | balance | 88 |
| | D6 | 0.2 | C:5 | 18 | 5 | balance | 83 |
| | D7 | 0.2 | C:2 | 18 | 5 | balance | 76 |
| | D8 | 0.2 | C:10 | 18 | 5 | balance | 85 |
| | D9 | 0.2 | C:5 | 10 | 5 | balance | 82 |
| | D10 | 0.2 | C:5 | 25 | 5 | balance | 77 |
| | D11 | 0.2 | C:5 | 18 | 3 | balance | 90 |
| | D12 | 0.2 | C:5 | 18 | 10 | balance | 81 |
| | D13 | 0.03 | - | 18 | 3 | balance | 82 |
| | D14 | 0.2 | - | 18 | 10 | balance | 83 |
| | D15 | 2 | - | 18 | 10 | balance | 84 |
| Comparative Tests | D1 | - | C:5 | 13 | 5 | balance | 70 |
| | D2 | - | C:5 | 18 | 5 | balance | 72 |
| | D3 | - | C:5 | 25 | 5 | balance | 71 |
| | D4 | - | - | 18 | 3 | balance | 55 |
| | D5 | - | - | 18 | 10 | balance | 53 |

As is clear from Table 4, the hardened bodies formed from Tests D1 through D15 containing sodium pyrophosphate have even higher strength in comparison with the hardened bodies formed from Comparative Tests D1 through D5 that did not contain sodium pyrophosphate.

### INDUSTRIAL APPLICABILITY

The calcium phosphate cement of the present invention enables the forming of hardened materials having a high strength, and in addition to significantly contributing to improving the strength of treated reinforced bone, enables the amount of hardened material used to be reduced, thereby having industrially useful properties.

## Claims

1. A calcium phosphate cement containing 0.03 to 2 wt% of sodium pyrophosphate and 3 to 35 wt% of dibasic calcium phosphate, the remainder being at least one type selected from the hydration reaction product of dibasic calcium phosphate and α-tribasic calcium phosphate, tetrabasic calcium phosphate and α-tribasic calcium phosphate, and unavoidable impurities.

2. The calcium phosphate cement according to claim 1 that contains 0.03 to 0.5 wt% of sodium pyrophosphate, 3 to 10 wt% of dibasic calcium phosphate, and 10 to 25 wt% of tetrabasic calcium phosphate, the remainder being α-tribasic calcium phosphate and unavoidable impurities.

3. The calcium phosphate cement according to claim 1 that contains 0.03 to 1.5 wt% of sodium pyrophosphate and 3 to 25 wt% of dibasic calcium phosphate, the remainder being α-tribasic calcium phosphate and unavoidable impurities.

4. The calcium phosphate cement according to claim 1 that contains 0.03 to 2 wt% of sodium pyrophosphate and 5 to 35 wt% of dibasic calcium phosphate, the remainder being tetrabasic calcium phosphate and unavoidable impurities.

5. The calcium phosphate cement according to claim 1 that contains 0.03 to 2.0 wt% of sodium pyrophosphate, 2 to 10 wt% of the hydration reaction product of dibasic calcium phosphate and α-tribasic calcium phosphate, 3 to 10 wt% of dibasic calcium phosphate, and 10 to 25 wt% of tetrabasic calcium phosphate, the remainder being α-tribasic calcium phosphate and unavoidable impurities.
